(19) **Europäisches Patentamt / European Patent Office / Office européen des brevets**

(11) **EP 4 321 616 A1**

(12) **EUROPEAN PATENT APPLICATION**

(43) Date of publication:
**14.02.2024 Bulletin 2024/07**

(21) Application number: **22189534.5**

(22) Date of filing: **09.08.2022**

(51) International Patent Classification (IPC):
*C12N 9/18* (2006.01)       *C12N 9/20* (2006.01)
*C12N 11/093* (2020.01)      *C12N 11/096* (2020.01)
*C12N 9/96* (2006.01)        *C08J 11/10* (2006.01)
*C12N 11/14* (2006.01)

(52) Cooperative Patent Classification (CPC):
**C12N 9/18; C08J 11/105; C12N 9/20; C12N 9/96; C12N 11/093; C12N 11/096; C12N 11/14; C12Y 301/01003; C12Y 301/01074;** C08J 2367/02; C08J 2367/04; C08J 2375/06; C12R 2001/645

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Covestro Deutschland AG**
  **51373 Leverkusen (DE)**
• **Alma Mater Studiorum - Università di Bologna**
  **40126 Bologna (IT)**

(72) Inventors:
• **Thiebes, Christoph**
  **51373 Leverkusen (DE)**
• **Fait, Thomas**
  **51373 Leverkusen (DE)**
• **Sisti, Laura**
  **40067 Rastignano (IT)**

• **Zanaroli, Giulio**
  **40136 Bologna (IT)**
• **Totaro, Grazia**
  **44122 Ferrara (IT)**
• **Romano, Angela**
  **40134 Bologna (IT)**
• **Rosato, Antonella**
  **10128 Torino (IT)**
• **Celli, Annamaria**
  **47122 Forli (IT)**

(74) Representative: **Levpat**
**c/o Covestro AG**
**Gebäude K12**
**51365 Leverkusen (DE)**

Remarks:
The complete document including Reference Table(s) and the Sequence Listing(s) can be downloaded from the EPO website

(54) **THERMALLY STABLE CARBOXYLIC ESTER HYDROLASES IN LAYERED DOUBLE HYDROXIDES FOR INTRINSIC RECYCLABLE POLYMERS**

(57)  The present invention relates to the stabilization of lipases in layered double-hydroxide structures, polymers containing such stabilized lipases and methods for manufacturing enzymatically degradable polymers.

**EP 4 321 616 A1**

**Description**

The present invention relates to the stabilization of carboxylic ester hydrolases in layered doublehydroxide structures, polymers containing such stabilized carboxylic ester hydrolases and methods for manufacturing enzymatically degradable polymers.

As of today a large proportion of plastic materials cannot be recycled at their end of life but have to be burned or deposited in landfills. If plastics are not properly disposed of, they accumulate in the environment, e.g. in the world's streams and oceans. Since the manufacture of polymers requires energy and most of the presently used materials are based on petrochemicals, the single use of plastic materials contributes to global $CO_2$-emissions. Thus, it is desirable to recycle used plastic materials in order to reduce $CO_2$-emissions and waste.

One obstacle to this aim is the fact that many plastic materials are not used as pure materials but as mixtures of several compounds with different properties. This makes recycling efforts difficult and often economically infeasible. One example are multilayer materials used for the packaging of food or beverages: in order to fulfill the different functions required such as retention of moisture, protection against UV-radiation or oxygen, several sheets of different polymers are bonded by adhesives to form a laminate which combines all the desired properties. The separation of the different layers at the end of life ranges from difficult to impossible.

One attempt to overcome this problem is the use of enzymatically degradable plastics or adhesives, in the case of multilayer structures, comprising the degrading enzyme. The enzymatic degradation of the adhesive leads to the separation of the different sheets of plastic, thus facilitating the recycling of the separate materials. Unfortunately, enzymes are in many cases temperature sensitive so that they do not remain active at those elevated temperatures which are typically employed for the processing of multilayer materials.

Some solutions for this problem have been tried. It is possible to use genetic engineering to create new thermo-stable variants of polymer-degrading enzymes (US 2019225954; WO 2020/021116; WO 2020/021117; WO 2020 021118; WO 2018/109183). It has also been tried to use complex multi-step processes at different temperatures in which the enzyme is embedded with a carrier such as the arabic gum in an aqueous formulation with a low melting polymer as PCL and the melt is then mixed with a second polymer, such as PLA, at high temperature (WO 2019/043134; CA 3 072 865; WO 2016/198652). However, it would be desirable to have a system which can be used with existing non-modified enzymes and which does not require manufacturing processes of increased complexity. The most desirable solution would be an enzyme which can simply be added to conventional plastics or adhesive compositions for use in conventional manufacturing processes. This problem is solved by the embodiments defined in the description below and the claims.

Hence, in a first embodiment, the present invention relates to a hydrolase of the enzyme class EC 3.1.1.74 (cutinases) or EC 3.1.1.3 (triacylglycerol lipases) immobilized in a layered double hydroxide structure (LDH).

**Cutinase**

The term "cutinase" encompasses all enzymes of EC class 3.1.1.74. These are hydrolases acting on carboxylic ester bonds in cutin. Preferably, the cutinase is the cutinase from *Humicola insolens* having an amino acid sequences as defined by SEQ ID NO.: 1 or a variant thereof.

**Triacylglycerol lipase**

The term "triacylglycerol lipase" refers to all enzymes in EC class 3.1.1.3. These are hydrolases acting on carboxylic ester bonds in triglycerides. Preferably, the triacylglycerol lipase is the triacylglycerol lipase B from *Moesziomyces antarcticus* (previously named *Candida antarctica* and *Pseudozyma antarctica*) as defined by SEQ ID NO.: 2 or a variant thereof.

Both classes of enzymes will in the following also be referred to as "carboxylic ester hydrolase" or "carboxylic ester hydrolases". Thus, unless otherwise explicitly stated, the term "carboxylic ester hydrolase" will refer to the cutinases and/or triacylglycerol lipases defined above.

**Variant**

The term "variant" when referring to one of the carboxylic ester hydrolases of the present invention relates to proteins having at least 90 %, more preferably at least 95 % and most preferably at least 98 % sequence identity to the amino acid sequence defined by SEQ ID NO.: 1 or SEQ NO.: 2, provided that such proteins still have carboxylic ester hydrolase activity.

The person skilled in the art is aware that additions or deletions of amino acids from SEQ ID NO.: 1 or SEQ NO.: 2 may shift the particular amino acids positions recited in this application. Therefore, any amino acid position referred to in this application based on the wild-type sequence must be understood as referring to the homologous amino acid position in a protein derived from SEQ ID NO.: 1 or SEQ NO.: 2 by deleting or adding amino acids.

Variants of SEQ ID NO.: 1 or SEQ NO.: 2 having the degrees of sequence identity set forth above are preferably derived from SEQ ID NO.: 1 or SEQ NO.: 2 only by conservative substitutions of amino acids.

A "conservative substitution" is a substitution on one amino acid by a different amino acid with similar properties. Preferably, it is an exchange of an amino acid with a non-polar side chain for another amino acid with a non-polar side chain, an exchange of an amino acid with an acidic side chain for another amino acid with an acidic side chain, an amino acid with a basic side chain for another amino acid with a basic side chain or an exchange of an amino acid with a polar side chain for another amino acid with a polar side chain. Because the properties of the side chains in conservative substitutions do not change much, the overall structure of the resulting protein will not be severely affected.

Variants of SEQ ID NO.: 1 or SEQ NO.: 2 derived from this sequence by addition of amino acids and having the degrees of sequence identity set forth above are, preferably, derived from SEQ ID NO.: 1 or SEQ NO.: 2 by addition of up to 35, more preferably up to 20 and most preferably up to 10 amino acids at the C-terminus and/or the N-terminus. Typical additions to a protein are additions of amino acid sequences which make the purification of the expressed protein easier. One particularly preferred modification is the addition of several histidines, a so-called "his-tag". Also preferred is the addition of peptide linkers.

Variants of SEQ ID NO.: 1 or SEQ NO.: 2 derived from this sequence by deletion of amino acids and having the degrees of sequence identity set forth above are, preferably, derived from SEQ ID NO.: 1 or SEQ NO.: 2 by deletion of up to 35, more preferably up to 20 and most preferably up to 10 amino acids at the C-terminus and/or the N-terminus.

"Polar amino acids" or "amino acids with polar side chains" as understood by the present application are glycine, serine, threonine, cysteine, asparagine, glutamine, tryptophan and tyrosine.

"Non-polar amino acids" or "amino acids with non-polar side chains" as understood by the present application are alanine, valine, leucine, iso-leucine, phenylalanine, proline, and methionine.

Amino acids with acidic side chains as understood by the present application are aspartate and glutamic acid.

Amino acids with basic side chains as understood by the present application are lysine, arginine and histidine.

The presence of carboxyl ester hydrolase activity is preferably determined by a spectrophotometric assay using 4-nitrophenyl butyrate (4-NPB) as substrate. A volume of 0.1 mL of each sample is added to 3 mL of sodium phosphate buffer (0.1 M, pH 7.8) containing 1 mM of 4-NPB. In the presence of the enzyme, this substrate is rapidly hydrolyzed into butyric acid and 4-nitrophenol, whose formation can be spectrophotometrically measured at 420 nm.

**Layered double hydroxides (LDH)**

The term "layered double hydroxides" in general is known to the Person Skilled in the Art. Structurally, LDH is a natural/synthetic clay adopting a structure derived from brucite $Mg(OH)_2$ with layers built from octahedra in which the center $Mg^{2+}$ is surrounded by six hydroxyl groups. The isomorphous substitution of some divalent cations by trivalent cations provokes an excess of positive charges, counter balanced by some interlayer anions and water leading to a neutral structure.

Preferably, the LDH comprises at least one cation selected from the group consisting of $Mg^{2+}$, $Ni^{2+}$, $Zn^{2+}$, $Co^{2+}$, $Ca^{2+}$ and $Cu^{2+}$ and at least cation selected from the group consisting of $Al^{3+}$, $Ga^{3+}$, $Fe^{3+}$ and $Cr^{3+}$. It is more preferred that the LDH comprises a combination of $Mg^{2+}$ and $Al^{3+}$. I.e. the presence of both a divalent and a trivalent cation is essential.

In a preferred embodiment of the present invention, the LDH is defined by formula (I)

$$[M^{II}_{1-x}M^{III}_{x}(OH)_2]^{x+}(Y^{n-})_{x/n}\, mH_2O \qquad (I)$$

wherein

Y represents a carboxylic ester hydrolase;

m represents a number in the range from 0 to 2;

$M^{II}$ represents one or more divalent cations selected from the group consisting of $Mg^{2+}$, $Zn^{2+}$, $Co^{2+}$, $Ni^{2+}$, $Ca^{2+}$, and $Cu^{2+}$;

$M^{III}$ represents one or more trivalent cations selected from the group consisting of $Al^{3+}$, $Ga^{3+}$, $Fe^{3+}$ and $Cr^{3+}$;

x represents a number, $0<x<1$, preferably $0.1<x<0.5$;

**Immobilization**

The term "immobilized" refers to a state of the carboxylic ester hydrolase, where it is bound within the LDH in such a way that it cannot be separated from the LDH by simple washing. Importantly, the carboxylic ester hydrolase activity of the immobilized carboxylic ester hydrolase is very low and preferably absent and that immobilized carboxylic ester hydrolases are very thermostable. Carboxylic ester hydrolase activity can be restored by using an ion exchange medium comprising anions which substitute the carboxylic ester hydrolase in the positively charged layers surroundings of the LDH.

Thus, the immobilization advantageously provides carboxylic ester hydrolases in a state, where they can be added to polymers or polymerizable compositions in a largely inactive but activable state. Thus, during the life time of the product the carboxylic ester hydrolase does not negatively affect its properties, particularly the integrity of the polymer. Only when the carboxylic ester hydrolase is activated, it breaks down the polymer.

Immobilization of the carboxylic ester hydrolase within the LDH is preferably achieved using the coprecipitation method. Alternative methods for LDH synthesis are: ion exchange, hydrothermal treatment and reconstruction. The first method involves the use of a LDH precursor, whose interlayer anions (i.e. chloride, nitrate, carbonate) are replaced by the anions from a specific solution through an ion-exchange mechanism. A hydrothermal preparation is based on the treatment of freshly precipitated mixed hydroxides or mechanical mixtures of the oxides with water (possibly in the presence of other anions) at a temperature higher than 100 °C, under pressure in an autoclave (Cavani et al. Catalysis Today, 11, 1991, 173-301). Another frequently employed method is the reconstruction from a calcined precursor. This calcined product (a mixture of mixed oxides of metals) can be transformed into the original layer structure (in presence of water and the anion to be interleaved) thanks to the structural memory effect. The reconstruction of LDHs after the calcination is generally believed to be a rehydration process from the mixed oxides to bimetallic hydroxide (Gao et al. Langmuir 2018, 34, 19, 5386-5395). The coprecipitation route is preferable, being a "soft chemistry" tunable process, allowing to select variable conditions (pH, temperature, solvent, reagents concentration). It consists in the slow coprecipitation of an aqueous solutions of the metal salts within the solution containing the molecule to be interleaved. These negatively charged groups can then interact with the cations of the LDH. It is preferred that the molar ratio of hydrolase to Al cation in the LDH is between 3.4 : 1 to 0.5 : 1. A preferred molar ratio is between 2.8 : 1 and 1.5 : 1. It is preferred to use a first solution comprising the metal salts and to adjust the pH of this solution to a pH above the isoelectric point of the carboxylic ester hydrolase, preferably pH 10 by adding a suitable base, preferably NaOH. This solution is then added to a second solution comprising the carboxylic ester hydrolase. The pH in the mixture must be kept above the isoelectric point of the carboxylic ester hydrolase. This can be achieved by adding a suitable base as described above.

The immobilized carboxylic ester hydrolase is thermally-stable, i.e. it may be subjected to increased temperatures without inacceptable loss of activity. Preferably, it can be heated for up to 120 minutes to up to 90 °C or for up to 180 seconds to up to 150 °C without losing more than 50 % of its activity. A heating for up to 10 seconds to up to 200 °C is also possible without loss of more than 50 % of the original activity. Reference for these values is the activity of a carboxylic ester hydrolase which is not heated to a temperature above 40 °C.

In a preferred embodiment of the present invention, the surface of the immobilized carboxylic ester hydrolase is covered with a surfactant. In principle, anionic, cationic, zwitterionic or non-ionic surfactants are equally suitable. Preferred surfactants are selected from the group consisting of soy lecithin, cetyltrimethylammonium chloride (CTAC), sodium oleate, sodium stearate and stearic acid. However, in a more preferred embodiment, the surfactant is an anionic surfactant, most preferably sodium stearate. The use of surfactant is particularly useful for embedding the immobilized carboxylic ester hydrolases in hydrophobic polymers, especially polyesters and polyester-polyurethanes.

**Polymer comprising the immobilized carboxylic ester hydrolase**

In another embodiment, the present invention relates to a polymer comprising an immobilized carboxylic ester hydrolase as defined above, wherein the polymer comprises ester groups.

As the carboxylic ester hydrolase can be activated by releasing it from the LDH using methods described below, the polymer comprising the carboxylic ester hydrolase is enzymatically degradable. I.e. the activated carboxylic ester hydrolase is capable of hydrolyzing at least a part of the ester bond comprised by the polymer. This decreases the molecular weight of the polymer. This process may release defined compounds of low molecular weight which can be used as building blocks for the synthesis of another polymer. However, if the polymer is an adhesive, a complete degradation is not required. In this application it is sufficient if the molecular weight of the polymer is sufficiently decreased to deprive the adhesive of its properties so that the parts connected originally by the adhesive can be mechanically separated.

In a preferred embodiment of the present invention, the polymer having ester groups and comprising an immobilized carboxylic ester hydrolase forms part of an adhesive joint or of an adhesive composition.

**Polymer**

The term "polymer" refers to all polymers comprising carbonic ester groups, particularly polyester polyurethanes and polyesters. As the cutinases and triacylglycerol lipases of the present invention have esterase activity, the polymer must comprise ester groups to be degradable.

Preferred polyesters are poly(butylene succinate) (PBS), poly(butylene succinate-co-adipate) (PBSA), poly(caprolactone) (PCL), poly(lactic acid) (PLA) and poly(propylene carbonate) (PPC). PBS is a semicrystalline polyester derived from 1,4 butanediol and succinic acid, both obtainable from sugar fermentation. PBSA is a semicrystalline copolymer of PBS, with adipic acid as comonomer, which is also potentially biobased. PCL is a semicrystalline polymer as well, prepared from the potentially biobased cyclic ester ε-caprolactone or 6-hydroxyhexanoic acid. PLA can be prepared from pure L-lactic or D-lactic isomers, or a racemic mixture of both, obtaining poly-L-lactic (PLLA) acid, poly-D-lactic acid (PDLA), or poly-D,L-lactic acid (PDLLA), respectively. The stereochemistry affects the material properties: PLLA is semicrystalline, while PDLLA is an amorphous polymer. PPC is a regular alternating amorphous copolymer prepared from $CO_2$ and the most reactive cyclic ether propylene oxide. Especially preferred polyesters for the current application are PBSA, PBS and PCL.

As understood in the present application polyester-polyurethanes are polymers that contain at least two ester and at least two urethane groups in the same molecule. A description of polyurethanes, including polyester-polyurethanes, their manufacturing and their use as adhesives and coatings is described in Leimenstoll/Stepanski, Polyurethanklebstoffe, Springer Vieweg Essentials, ISBN 978-3-658-12269-0 and Meier-Westhues et al, Polyurethanes, 2nd Edition, Vinzenz Hanover 2019, ISBN 33-86630-782-9. The skilled chemist is aware of the basic methods to produce such polymers, for example by reacting a polyester polyol, obtainable by polycondensation of polyacids and polyols with a polyisocyanate. Polyisocyanates are molecules that contain more than one isocyanate groups. The group of polyisocyanates includes diisocyanates that contain two isocyanate groups in one molecule. Polyols are molecules that contain more than one hydroxy group. Polyacids are molecules that contain more than one acid group. Polyester-based polyols that can be used in the scope of this invention are for example polyfunctional alcohols with a number average molecular weight (determined by gel permeation chromatography in accordance with DIN 55672-1:2016-03) between 400 and 4000 g/mol and a functionality between 2 and 5.

Polyester polyols can be based on aromatic or aliphatic polyacids and polyols. Examples for such polyester polyols are poly(ethylene adipate), poly(diethylene adipate), poly(hexylene-co-neopentyl adipate), poly(butylene adipate), poly(ethylene-co-propylene adipate), poly(hexylene adipate-coisophthalate), poly(hexylene ortophthalate) with OH terminal groups. Preferably, aliphatic polyesters are used, most preferably linear polyesters based on adipic acid and diethylene glycol and linear polyesters based on adipic acid and ethylene glycol.

Polyisocyanates that can be used to obtain polyester-polyurethanes are diisocyanates like 1,4-diisocyanatobutane (BDI), 1,5-diisocyanatopentane (PDI), hexamethylene 1,6-diisocyanate (HDI), 2-methyl-1,5-diisocyanatopentane, 1,5-diisocyanato-2,2-dimethylpentane, 2,2,4- or 2,4,4-trimethyl-1,6-diisocyanatohexane, 1,10-diisocyanatodecane, 1,3- and 1,4-diisocyanatocyclohexane, 1,4-diisocyanato-3,3,5-trimethylcyclohexane, 1-isocyanato-3,3,5 -trimethyl-5 -isocyanatomethyl-cyclohexane (isophorone diisocyanate; IPDI), 1,3-diisocyanato-2-methylcyclohexane, 1,3-diisocyanato-4-methylcyclohexane, 1-isocyanato-1-methyl-4(3)-isocyanatomethylcyclohexane, 2,4'- and 4,4'-diisocyanatodicyclohexylmethane (H12MDI), 1,3- and 1,4-bis(isocyanatomethyl)cyclohexane, bis(isocyanatomethyl)norbornane (NBDI), 4,4'-diisocyanato-3,3'-dimethyldicyclohexylmethane, 4,4'-diisocyanato-3,3',5,5'-tetramethyldicyclohexylmethane, 4,4'-diisocyanato-1,1'-bi(cyclohexyl), 4,4'-diisocyanato-3,3'-dimethyl-1,1'-bi(cyclohexyl), 4,4'-diisocyanato-2,2',5,5'-tetramethyl-1,1'-bi(cyclohexyl), 1,8-diisocyanato-p-menthane, 1,3-diisocyanatoadamantane, 1,3-dimethyl-5,7-diisocyanatoadamantane, 1,3- and 1,4-bis(isocyanatomethyl)benzene (xylylene diisocyanate; XDI), 1,3- and 1,4-bis(1-isocyanato-1-methylethyl)benzene (TMXDI), bis(4-(1-isocyanato-1-methylethyl)phenyl) carbonate, 2,4- and 2,6-diisocyanatotoluene (TDI), 2,4'- and 4,4'-diisocyanatodiphenylmethane (MDI), 1,5-diisocyanatonaphthalene and mixtures thereof. Further diisocyanates that are likewise suitable can additionally be found, for example, in Justus Liebigs Annalen der Chemie, volume 562 (1949) p. 75-136.

It is also possible to use oligomers of the above-described diisocyanates, like those comprising uretdione, carbodiimide, urethane, allophanate, isocyanurate, biuret, iminooxadiazindione and or oxadiazinetrione groups in their structure.

Furthermore isocyanate functional prepolymers that can be obtained from reacting such polyisocyanate and/or diisocyanates with polymers or oligomers comprising isocyanate reactive groups can be employed. Most preferably, isocyanate prepolymers based on MDI and TDI are used.

To the one skilled in the art there are many different ways known to obtain polyester-polyurethanes that are used for example as adhesives or coatings.

For example, an isocyanate functional prepolymer based on a polyester polyol can be cured with moisture (moisture curing 1-component system).

Another example to form high molecular weight polyester-polyurethanes is by mixing the two components of a 2-component (2C-) polyurethane system, where component A comprises molecules with an isocyanate group and component B comprises molecules with isocyanate reactive groups and subsequent reaction of the isocyanate groups with the isocyanate reactive groups to form a high molecular weight polymer, where component A and/or B comprise polyester groups. For example component A can comprise a polyisocyanate oligomer, preferably an urethane-containing oligomer of TDI or isocyanurate-containing oligomer of HDI, and component B can comprise a hydroxy-functional polyester polyol or hydroxy-functional polyester polyol that has been chain elongated with a diisocyanate (excess of hydroxy groups), or component A can comprises an isocyanate functional prepolymer and component B a low molecular weight polyalcohol like glycerol or trimethylolpropane. The preferred diisocyanate for chain elongation are MDI, TDI and HDI, most preferably HDI.

Other examples are physically drying systems, comprising high-molecular weight polyester-polyurethanes manufactured in solvents or water and are available in form of solutions or dispersions. They can be applied in liquid form as adhesives or coatings and solidify upon evaporation of the solvent or water.

Also combinations of these three principles are possible.

Examples of 1C, 2C and physically drying systems can be found in Meier-Westhues et al, Polyurethanes, 2nd Edition, Vinzenz Hanover 2019, ISBN 33-86630-782-9.

The immobilized carboxylic ester hydrolase may be mixed with the precursors of the polymers before synthesis of the polymer or it may be mixed with the synthetized polymer. Both variants result in a polymer comprising the immobilized and inactive carboxylic ester hydrolase. Thus, the polymer is stable until the carboxylic ester hydrolase is released from the LDH, so that degradation of the polymer can be triggered at will.

In a preferred embodiment of the present invention, the polymer is a polyester-polyurethane and the immobilized hydrolase is comprised in at least one of the liquid precursors of the cured high-molecular weight polyester-polyurethane. In this case the immobilized hydrolase is added to the A and/or B component of a 2C-System or to the 1C- or physically drying system before curing.

In a preferred embodiment of the present invention, the polymer comprises in addition to the immobilized carboxylic ester hydrolase organic or inorganic additives or fillers. Preferred additives are UV-stabilizers. Preferred inorganic fillers are calcium carbonate, clay, talcum, gypsum, aluminum trihydrate, kaolin, mica, wollastonite, glass fiber, aramid fiber, carbon fiber, and carbon black. Also fillers that can reduce the transmission of gases through the adhesive layer, like nano-clays can be used.

In an especially preferred embodiment of the present invention, the polymer having ester groups and comprising the immobilized carboxylic ester hydrolase is a part of a laminate. More preferably, it is incorporated/comprised in one of the adhesive layers that bond together two or more sheets of the laminate. The laminate is, preferably part of a food packaging or a beverage packaging.

**Method**

In yet another embodiment, the present invention relates to a method comprising the steps of

a1) Providing an immobilized carboxylic ester hydrolase as defined by any one of claims 1 to 5 and a polymer having ester groups; and

b1) Mixing the polymer having ester groups and the immobilized carboxylic ester hydrolase, so that a polymer comprising the immobilized carboxylic ester hydrolase is formed;

or

a2) Providing a crosslinkable composition comprising (i) a prepolymer containing carboxylic ester groups and additionally either isocyanate groups or groups that can react with NCO groups and (ii) the immobilized carboxylic ester hydrolase; and

b2) crosslinking the prepolymer (i) with a crosslinker containing either isocyanate-reactive groups or isocyanate-groups so that a polymer is formed from the polymerizable composition and said polymer comprises the immobilized carboxylic ester hydrolase.

The product resulting from this method is the polymer having ester groups and comprising an immobilized carboxylic ester hydrolase as defined above.

In the first embodiment (method steps a1 and b1), the polymer having ester groups and comprising the immobilized carboxylic ester hydrolase is provided by mixing the polymer with the immobilized carboxylic ester hydrolase. This embodiment is particularly preferred for polyesters comprising the immobilized carboxylic ester hydrolase and for physically drying polyester-polyurethane systems, comprising high-molecular weight polyester-polyurethanes which do not need chain extension by a crosslinker as described below.

In the second embodiment (method steps a2 and b2), the polymer having ester groups and comprising the immobilized carboxylic ester hydrolase is provided by providing (i) a prepolymer containing carboxylic ester groups and either isocyanate groups or groups that can react with NCO groups and (ii) the immobilized carboxylic ester hydrolase. If the prepolymer comprises isocyanate groups, the crosslinker preferably comprises isocyanate-reactive groups and *vice versa.* The prepolymer and the crosslinker are then crosslinked, preferably by forming urethane and/or urea groups to give the polymer. As the immobilized carboxylic ester hydrolase is present during polymerization, it will be embedded in the formed polymer network. This embodiment is particularly preferred for polyester polyurethanes comprising the immobilized carboxylic ester hydrolase. "Isocyanate-reactive groups" are preferably hydroxyl groups, amino groups and thiol groups. More preferably they are hydroxyl groups or amino groups.

A crosslinkable composition is a composition comprising at least the components described above in such amounts and such a form that a polymer can be formed from NCO-group containing substances and NCO-reactive groups containing substances (preferably OH-group containing substances) the isocyanate-terminated prepolymer and the crosslinker by crosslinking them.

Based on the temperature stability of the immobilized enzyme, any heating step during the method of the present invention may last up to 120 minutes if a temperature of 90 °C is not exceeded or up to 180 seconds if a temperature of 150 °C is not exceeded. A short heating spike of up to 10 seconds to up to 200 °C is also possible.

Preferably the prepolymer having carboxylic ester groups has a number average molecular weight between 1,000 and 15,000 g/mol. The average isocyanate functionality or the functionality of isocyanate-reactive groups is preferably between 1.5 and 4 per molecule.

The "crosslinker" is a low molecular weight compound having at least two isocyanate-groups or isocyanate-reactive groups per molecule. Preferably, the component B has a number average molecular weight between 80 and 2,000 g/mol.

In this context, the "prepolymer" is the molecule having the larger molecular weight and - conversely - the crosslinker is the molecule with the lower molecular weight. An NCO-terminated prepolymer is combined with a crosslinker having NCO-reactive groups, whereas a prepolymer having NCO-reactive groups is combined with an NCO-functional crosslinker.

It is preferred to use the immobilized carboxylic ester hydrolase and the polymer or the prepolymer and the crosslinker in amounts that result in a polymer containing 0.5 to 5.0 wt.-% of immobilized carboxylic ester hydrolase based on the total weight of polymer and immobilized carboxylic ester hydrolase. The mass of the immobilized carboxylic ester hydrolase is made up by the combined masses of carboxylic ester hydrolase and LDH.

In a preferred embodiment, the above-defined method additionally comprises a second set c):

c) Activating the immobilized carboxylic ester hydrolase.

The immobilized carboxylic ester hydrolase is activated by releasing it from the LDH. This is preferably done by contacting the polymer with a suitable ion exchange medium, thus releasing the carboxylic ester hydrolase in its active form. The active carboxylic ester hydrolase then splits at least some of the ester bonds of the polymer.

The activation occurs through an ion exchange mechanism in salt solution: the anions from the salt interact with the positive charged lamellae of the host structure and substitute the anionic enzyme, which can be released and recover its degrading activity. The release generally follows two steps, first a rapid release, and secondly a maintained, slow release, related to anionic exchange with anions of the medium. The anion exchange depends on the charges and the ionic radius, meaning that with the increase of charges and decrease of ionic radius, the exchangeability of the incoming anion increases. For example, the order for simple inorganic anions decreases as follow $CO_3^{2-}$ > $HPO_4^{2-}$ > $SO_4^{2-}$ for divalent anions, and $OH^-$ > $F^-$ > $Cl^-$ > $Br^-$ > $NO_3^-$ > $I^-$ for monovalent anions. Especially suitable as ion exchange media in the method of the present invention are sodium phosphate buffer (0.1 M, pH 8), citrate-phosphate buffer (0.1 M, pH 8), sodium sulfate (0.08 M, pH 7.5), potassium carbonate (0.1 M, pH 11.5) and sodium chloride (1 M, pH 7.4). More preferred are sodium sulfate (0.08 M, pH 7.5) and sodium phosphate buffer (0.1 M, pH 8). A particularly preferred ion exchange medium to be used according to the present invention is sodium phosphate buffer (0.1 M, pH 8)

**Laminate**

In another preferred embodiment, the present invention relates to a laminate comprising the polymer defined above. The laminate is, preferably, a multilayer structure comprising a first layer which is bonded to another layer by the polymer of the present invention which comprises a carboxylic ester hydrolase. Preferably, the "layers" consist of materials selected from the group consisting of card board, metal, wood and plastic. Most preferably the layers consist of metal foil or plastic foil.

**Use of the immobilized carboxylic ester hydrolases**

In yet another embodiment, the present invention relates to the use of the immobilized carboxylic ester hydrolases for the degradation of polymers comprising ester groups. This use involves the provision of a polymer having ester groups and comprising an immobilized carboxylic ester hydrolase as described above. The actual degradation is then triggered by exposing the polymer with immobilized carboxylic ester hydrolase to a suitable ion exchange medium as described above, thus releasing the carboxylic ester hydrolase in its active form.

**Kit of parts**

In yet another embodiment, the present invention relates to a kit of parts comprising

a) An immobilized carboxylic ester hydrolase as defined above in this application; and

b)

(i) in one container a prepolymer containing carboxylic ester groups and additionally either isocyanate groups or groups that can react with NCO and in a second container a crosslinker containing either isocyanate-reactive groups or isocyanate-groups or

(ii) a polymer having ester groups.

All definitions given above for the polymer also apply to this embodiment.

The term "kit of parts" refers to collection of the separate constituents of the kit in such a form that they are storable and can be joined by mixing. The kit may additionally contain instructions on its use, particularly on the mixing ratio of the constituents and the application of the mixture for its purpose.

In a preferred embodiment, the kit of parts is an adhesive composition or can be used to form an adhesive composition by mixing two or more of its constituents. An "adhesive composition" is a composition which can be used to form an adhesive joint. The adhesive Joint will then comprise the polymer and the immobilized carboxylic ester hydrolase. It is, thus, enzymatically degradable upon activation of the carboxylic ester hydrolase.

In yet another embodiment, the present invention relates to the use of the above-defined kit for providing an enzymatically degradable polymer.

The following examples are merely intended to illustrate the invention. They shall not limit the scope of the claims in any way.

**Examples**

**1. EXPERIMENTAL**

**1.1 Materials**

Cutinase from *Humicola insolens* (product Novozym$^©$51032) was provided by ChiralVision as a solution (concentration of 20 mg/mL). Sodium phosphate dibasic ($Na_2HPO_4$), sodium phosphate monobasic ($NaH_2PO_4$), 4-nitrophenyl butyrate (4-NPB, $\geq$98%), sodium hydroxide (NaOH), sodium carbonate ($Na_2CO_3$), copper sulfate ($CuSO_4$), bovine serum albumin (BSA), sodium tartrate ($C_4H_4Na_2O_6$), 2N Folin & Ciocalteu's phenol reagent, aluminum nitrate $Al(NO_3)_3 \cdot 9H_2O$ and magnesium nitrate $Mg(NO_3)_2 \cdot 6H_2O$ were purchased from Merck (St. Louis, Missouri, USA). Potassium carbonate ($K_2CO_3$), sodium chloride (NaCl) and sodium sulfate ($Na_2SO_4$) were purchased from Carlo Erba Reagents. A commercial copolyester, namely poly(butylene succinate-co-adipate) (PBSA, commercial name bioPBS FD92PM), having the following composition $(PBS)_{0.7}$-$(PBA)_{0.3}$ was supplied by PTT MCC Biochem Company. Pural Mg61 from Sasol, supplied by Nachmann Srl (Italy), was used as commercial carbonate reference. Desmodur$^®$ L 75 (Covestro), Aromatic polyisocyanate based on toluene diisocyanate, 75% in ethyl acetate; NCO content of 13.3% by weight. All the materials were used as received.

**1.2 Immobilization of Cutinase on LDH**

All the LDHs were prepared by a slow coprecipitation. The molar ratio enzyme/$Al^{3+}$ was evaluated by considering the average molecular weight of the amino acidic sequence (122 g/mol), whose enzymes are made of. Briefly, $Mg(NO_3)_2 \cdot 6H_2O$ (0.209 g, $8.16 \times 10^{-4}$ mol) and $Al(NO_3)_3 \cdot 9H_2O$, (0.153 g, $4.08 \times 10^{-4}$ mol) in molar ratio 2/1, were mixed with deionized and decarbonated water (50 mL) and added dropwise to a water solution (100 mL) containing cutinase (5.6 mL, 20 mg/mL), in a molar ratio 2.2/1 respect to $Al^{3+}$. The initial pH in 100 mL of $H_2O$ was 5.7. NaOH solution (0.049 M) was added to reach a pH of 10. The coprecipitation was carried out during 3 h, with stirring, under nitrogen flow in order to avoid the presence of carbonate as competing ion. The pH was kept constant (10 $\pm$ 0.4) with the addition of NaOH solution (0.049 M). After the coprecipitation, the reaction mixture was aged for 3 h at room temperature (TA). The solid product was filtrated on Büchner, washed with 300 mL of deionized and decarbonated water (in small aliquots of 50 mL), dried overnight in desiccator, and then in vacuum-oven for 2 h at TA. The product was light yellow. The final reaction medium and the washing solution were collected to measure the amount and activity of the residual enzymes.

LDH are labeled LDH/Cut-X, where X is the enzyme ratio respect to $Al^{3+}$.

A sample of LDH/Cut-2.2/1 prepared by slow coprecipitation (3 h) followed by 1 h of ageing was subjected to a further surface treatment of 2 h with a 6 wt% water solution of sodium stearate as surfactant. This sample, labelled LDH/Cut-2.2/1$_{surf}$ was used to prepare polyurethane-formulations (see paragraph 1.7).

Details of all syntheses are reported in **Table 1.**

**Table 1:** LDHs with Cutinase prepared.

| Code | Mg/Al mol ratio | Enzyme loading (g)[a] | Enzyme loading ( mol)[b] | Enzyme/Al ( molar ratio) |
|---|---|---|---|---|
| LDH/Cut-3.4/1 | 2/1 | 0.168 | $1.38 \times 10^{-3}$ | 3.4/1 |
| LDH/Cut-2.8/1 | 2/1 | 0.139 | $1.14 \times 10^{-3}$ | 2.8/1 |
| LDH/Cut-2.2/1 | 2/1 | 0.111 | $9.10 \times 10^{-4}$ | 2.2/1 |
| LDH/Cut-1.0/1 | 2/1 | 0.052 | $4.26 \times 10^{-4}$ | 1.0/1 |
| LDH/Cut-0.5/1 | 2/1 | 0.027 | $2.21 \times 10^{-4}$ | 0.5/1 |

[a] Evaluated through the Lowry method;

[b] Enzyme average molecular weight calculated considering the amino acidic sequence.

## 1.3 Determination of immobilization efficiency and activity

The amount of enzyme is determined by measuring the initial and final concentration of enzyme in the medium using the Lowry method (Lowry, O. H., Rosebrough, N. J., Farr, A. L., & Randall, R. J. (1951). Protein measurement with the Folin phenol reagent. Journal of biological chemistry, 193, 265-275). The enzyme in the washing waters is also taken into consideration. More in detail, each sample (0.5 mL) was boiled for 1 minute, rapidly cooled on ice and supplemented with 2.5 mL of a reagent mixture consisting in 2% $Na_2CO_3$ (in NaOH 0.1N), 1% $CuSO_4$ (in dionex water), and 2% $C_4H_4Na_2O_6$ (in dionex water), in 100:1:1 ratio. After the reagent addition, the samples were immediately well mixed and incubated for exactly 10 minutes. A volume of 0.25 mL of Folin and Ciocalteu's phenol reagent (IN) was then added to each sample, which was immediately vortexed. After 30 minutes of incubation in dark at TA, the absorbance of each sample was then measured at 540 nm by means of a UV-Vis spectrophotometer (Varian Cary 100 bio, Dual Beam). The enzyme concentration was calculated through a calibration curve prepared using bovine serum albumin (BSA) dilution series (1 to 50 mg/L of BSA) as standards; the calibration curve was constantly verified.

The immobilization efficiency of the enzyme was calculated as following:

$$Immobilization\ efficiency\ (\%) = \frac{A-B}{A} * 100$$

A = total protein amount in the initial solution;
B = protein amount in final and washing solutions.

The enzyme activity was simultaneously checked by a continuous spectrophotometric assay using 4-nitrophenyl butyrate (4-NPB) as substrate. More in detail, a volume of 0.1 mL of each sample was added to 3 mL of sodium phosphate buffer (0.1M, pH 7.8) containing 1 mM of 4-NPB. In the presence of the enzyme, this substrate is rapidly hydrolyzed into butyric acid and 4-nitrophenol, whose formation can be spectrophotometrically measured at 420 nm over time. In particular, the absorbance ($Abs_{420nm}$) was continuously monitored until constant using a UV-Vis spectrophotometer set at 25 °C, and the absorbance per minute was obtained using the maximum linear rate. One unit of enzyme is defined as the amount of enzyme that hydrolyses 1 $\mu$mol of 4-nitrophenyl butyrate to butyric acid and 4-nitrophenol per minute under the assay conditions.

The activity of the LDH-immobilized enzyme was evaluated by adding a certain amount of LDH/Cut-2/1 powder to 3 mL of sodium phosphate buffer solution containing the 4-NPB substrate (1 mM); the absorbance at 420 nm was continuously measured using the spectrophotometer thermostated at 25 °C and the enzymatic activity defined as above.

## 1.4 Release kinetic experiments

A preliminary test was carried out on sample LDH/Cut-2.2/1 in both water and $Na_2SO_4$ solution (0.08M pH 7.5). Briefly, a known amount of LDH/Cut-2.2/1 (approximately 50 mg) was incubated into 10 mL of water, and 10 mL of sulfate solution. At specific times, both suspensions were centrifuged (5000 rpm, TA, 5min) and a small aliquot of the supernatants was recovered for the measurements of the enzymes amount and activity during 12 h.

Release tests of all the other samples were performed by incubating approximately 50 mg of LDH/enzyme into 10 mL of $Na_2SO_4$ solution (0.08M pH 7.5). After 12 h, the suspensions were centrifuged (5000 rpm, TA, 5min) and the supernatants were recovered for the measurements of the protein amount and activity recovery after release using the Lowry and the 4-NPB method, respectively, as described above.

Moreover, different media were tested, namely phosphate and citrate-phosphate buffers (0.1M, pH 8), potassium carbonate (0.1M, pH 11.5), and sodium chloride (1M, pH 7.4). 5 mg of LDH were put into 1 mL of different solutions. After 4 h, the suspensions were centrifuged (12000 rpm, TA, 2 min) and the supernatants were recovered for the measurements of the enzymes amount released and their relative activity.

### 1.5 Half-life and Thermal resistance

To investigate the thermal stability of free enzyme, small aliquots of enzyme was exposed to 90 °C for different times, quickly cooled on ice and assay for their residual activity. Tests have been performed in a thermostatic bath: 100 $\mu$L of enzyme solution was added to screw-cap glass vials containing 0.1M sodium phosphate buffer (5 mL). The residual activity [A] was measured by p-NPB assay and compared to the initial one [$A^0$]. Half-life of enzymes was calculated according to a second order kinetic ($r^2$ = 0.99) as follows:

$$t_{1/2} = \frac{1}{(k \times [A^0])}$$

where k is the kinetic constant, i.e., slope obtained by plotting 1/[A] versus the time; $A^0$ is the initial enzyme activity (Zanaroli et al. (2011). Selection of commercial hydrolytic enzymes with potential antifouling activity in marine environments. Enzyme and microbial technology, 49(6-7), 574-579).

To evaluate the thermal stability of LDH-immobilized enzymes, small aliquots of LDH/Cut-2.2/1 powder were subjected to different thermal treatments: 70 °C for 35 seconds; 90 °C for several minutes; 150 °C (180 seconds) and 200 °C (10 seconds); 105 °C (180 seconds) and 200 °C (10 seconds). The second temperature was chosen to evaluate the immobilized enzyme half-life, while the others were chosen to simulate industrial processes (such as dry/wet lamination or film extrusion), in order to have an idea of the performance at industrial scale. After the thermal treatment, the samples were quickly cooled on ice and their residual enzymatic activity was evaluated by p-NPB assay after release in 1 mL of $Na_2SO_4$ solution (0.08M) and compared to a control sample, used as reference. Tests have been performed in oven: around 5 mg of sample were put in oven paper in order to favor an immediate heating of the powder.

### 1.6 Evaluation of the degradation of a formulated polyester/LDH-immobilized enzyme

PBSA films with 1.2.5 and 5 wt % of immobilized cutinase (LDH/Cut-2.2/1) were prepared by compression molding at 120 °C. The resulting film thickness was 150 ($\pm$30) $\mu$m. The obtained formulated films were cut into several pieces and incubated in sodium phosphate buffer (0.1 M, 9 mL) under the optimal reaction condition of cutinase (pH 8, 40 °C). Their weight loss was investigated after 60 and 120 minutes of incubation. In addition, it was evaluated the release of cutinase from LDH structure in the liquid fraction, in terms of both protein concentration and enzymatic activity. Samples are labelled PBSA-LDH/Cut-X-Y%, where X is the enzyme ratio respect to $Al^{3+}$ and Y is the weight%.

### 1.7 Preparation of a formulated polyurethane/LDH-immobilized enzyme

A polyol based on HDI and a linear polyester based on adipic acid and diethylene glycol (OH-content 1.3% by weight) was prepared by reacting 0.91 g of HDI and 49.29 g of the polyester in a round bottomed flask for 4 h at 85 °C. After the reaction was complete a linear polyester polyurethane polyol with a OH-Nr of 30 mg KOH/g was obtained. 49.8 g of ethylacetate were added. Then, 1 g of the LDH-immobilized enzyme (LDH/Cut-2.2/1$_{surf}$ subjected to the surface treatment with sodium stearate) was incorporated into 89.89 g of the mixture by mixing with a stirrer.90.89 g of the LDH containing solvent borne polyester polyurethane were thoroughly mixed with Desmodur® L 75 (9.11 g) at 23 °C by using a stirrer and a film was prepared by coating with a doctor knife 20 $\mu$m onto a substrate. Optionally 0.1 g of Dioctyldilauryltin can be used as a possible catalyst to accelerate the reaction. The film was cured for 7 days at 23°C at 50% relative humidity (sample code PUR-SAMPLE 62-8).

In an analogous manner another crosslinked polyester polyurethane film was prepared by using 5 g of LDH-immobilized enzyme instead of 1g (sample code PUR-SAMPLE 62-2).

In an analogue manner another crosslinked polyester polyurethane film was prepared by using no enzyme. (Comparative example, sample code PUR-SAMPLE 62-1).

The obtained films were cut into small pieces and incubated in sodium phosphate buffer 0.1 M (3.5 mL) under the optimal reaction condition of cutinase (pH 8, 40 °C). Similarly, both formulations were incubated under vacuum at TA. The enzymatic degradation was investigated via weight loss test, after 24 h and 48 h. The test was conducted with five replicates of each formulation.

### 1.8 Characterization

Infrared spectra of sample powders were recorded using an ATR FT-IR over the wavenumber range 650-4000 $cm^{-1}$ using a Perkin Elmer Spectrum One FT-IR spectrometer equipped with a Universal ATR sampling accessory. Sixteen scans were taken for each spectrum at a resolution of 2 $cm^{-1}$.

Thermogravimetric analysis (TGA) was performed under air atmosphere for all samples, using a PerkinElmer TGA7 apparatus (gas flow 30 ml $min^{-1}$) at a 10 °C $min^{-1}$ heating rate from 50 to 800 °C. The 20% mass loss temperatures ($T_D{}^{20}$) and the residues were measured. To test cutinase, a lyophilized sample was used, obtained through a freeze-drying in skim milk solution.

The X-ray diffraction (XRD) analysis was carried out at room temperature by means of a Philips X-Pert Pro diffractometer. Data were acquired by exposing the samples to Cu-K$\alpha$ X-ray radiation. The patterns were collected with a step size of 0.03°, over 2$\theta$ range of 2.0-70°, with an accumulation time of 10 s per step.

### 2. RESULTS

### 2.1 Immobilization efficiency

The immobilization of Cutinase on Mg/Al layered double hydroxides (LDHs) through a classical coprecipitation procedure is investigated. Different enzyme loadings were tested: from 3.4/1 to 0.5/1, in terms of molar ratio respect to Al, calculated by considering a molecular weight of 122 g/mol for Cutinase, averaged from the amino acid sequence. This, in order to evaluate the optimal loading, with the highest immobilization efficiency. The ratio Mg/Al was 2/1, which is reported to maximize the charge density of the layers, favoring the enzyme intercalation (Bruna et al. (2019). Assembly of nitroreductase and layered double hydroxides toward functional biohybrid materials. Journal of colloid and interface science, 533, 71-81).

The amount of immobilized enzyme, or immobilization efficiency, was indirectly determined by measuring the initial and final concentration of enzyme in the reaction medium and in the washing solutions using the Lowry method. The results are reported in **Table 2.** As it can be observed, the immobilization efficiency is very high and quite constant, around 80%, in the range 2.2/1-0.5/1, while higher loadings seem to inhibit in part an effective immobilization **(Table 2).** The initial and final activity of the enzyme in the reaction medium and the activity in the washing solutions was simultaneously checked by the p-NPB assay (data not shown) and it is in agreement with the protein content (immobilization efficiency).

**Table 2:** Summary of the results obtained in terms of enzyme immobilization and activity recovery after release in $Na_2SO_4$ (0.08M).

| Sample | Immobilization efficiency (%) | After 12 h release in $Na_2SO_4$ (0.08M) | |
| --- | --- | --- | --- |
| | | Protein amount (%) | Total activity recovery (%) |
| LDH/Cut-3.4/1 | 41 | 42 | 41 |
| LDH/Cut-2.8/1 | 49 | 50 | 50 |
| LDH/Cut-2.2/1 | 80 | 67 | 67 |
| LDH/Cut-1.0/1 | 84 | 31 | 24 |
| LDH/Cut-0.5/1 | 81 | 29 | 8 |

The presence of Cutinase in the LDH structure was checked by infra-red spectroscopy. All LDHs spectra show the characteristic lattice vibration bands at low region ($\nu_{M-O}$ and $\nu_{O-M-O}$). A wide absorption band appears at 3300 $cm^{-1}$, which is due to NH and OH stretching of hydroxyl group and water molecules present in the interlayer space of LDHs. A sharp amide I peak appears at 1640 $cm^{-1}$, which is mainly due to the C=O stretching vibrations, together with a sharp amide II band at 1530 $cm^{-1}$, due to the bending modes of the N-H group and the stretching mode of the C-N group. The intensity of carbonate stretching at 1360 $cm^{-1}$ is lower respect to amide I and amide II, this confirms the fact that cutinase is present and carbonate is not predominant when the enzyme ratio is 2.2/1. Higher and lower loadings seem to contain more carbonate respect to cutinase, by observing the relative intensities of such bands. Therefore, cutinase is successfully immobilized on LDH, as well as carbonate ion in varying amount.

X-ray diffraction analysis confirms the results obtained by FT-IR. The crystallographic data are reported in **Table 3.** The reference sample profile exhibits the characteristic reflections of LDH materials with a series of (001) peaks appearing as narrow, symmetric, strong lines at $2\theta$-low angle, corresponding to the basal reflections, and the reflection (110) at higher $2\theta$, indicating an intra-layer structural ordering (profiles not shown). All the LDHs contain carbonate but large halos are present and the maxima of 003 and 006 reflections are shifted to slightly higher $2\theta$ values, as observable from $d_{003}$ data in Table 3: the system is somehow disturbed by cutinase, which is most likely adsorbed on the surface LDH, rather than intercalated between the lamellae. Lower enzyme loadings (1.0/1 or 0.5/1) present profiles more defined, according to the fact that more carbonate should be present.

In general, it can be concluded that immobilized systems were here obtained. However, the immobilization should be quite strong, as suggested by the fact that the "saw-toothed" reflections (110) and (113) tend to merge in all compositions especially 2.2/1. Such merging is due to a shift to lower $2\theta$ of (113), which is strongly I-dependent and generally caused by intercalation (Totaro et al. (2018). Dual chain extension effect and antibacterial properties of biomolecules interleaved within LDH dispersed into PBS by in situ polymerization. Dalton Trans., 47, 3155-3165).

**Table 3:** Cell parameters and thermal stability of the LDHs with Cutinase.

| Sample | $c'=d_{003}$ (Å)[a] | $c=3c'$ (Å)[b] | $d_{110}$ (Å)[c] | $a=2d_{110}$ (Å)[d] | $T_D^{20}$ (°C)[e] | Residue (%)[e] |
|---|---|---|---|---|---|---|
| Pural Mg61 | 7.6 | 22.8 | 1.52 | 3.04 | 343 | 57 |
| Cutinase | / | / | / | / | 156 | 3.1 |
| LDH/Cut-3.4/1 | 7.5 | 22.5 | 1.51 | 3.02 | 352 | 39 |
| LDH/Cut-2.8/1 | 7.6 | 22.8 | 1.51 | 3.02 | 323 | 34 |
| LDH/Cut-2.2/1 | 8.2 | 24.6 | 1.51 | 3.02 | 322 | 27 |
| LDH/Cut-1.0/1 | 7.6 | 22.8 | 1.51 | 3.02 | 304 | 37 |
| LDH/Cut-0.5/1 | 7.6 | 22.8 | 1.51 | 3.02 | 357 | 52 |

[a] c' is the interlayer distance and it is determined by the 003 reflection in XRD analysis.

[b] c is the total thickness of the brucite-like layers and the interlayer distance.

[c] determined by the 110 reflection in XRD analysis.

[d] *a* (lattice parameter) is related to the cation-cation distance.

[e] TGA under air flow (30 mL/min), samples were kept overnight under vacuum at TA before analysis; residue@800 °C.

Concerning the thermogravimetric experiments, data are reported in **Tables 3.** The commercial LDH (Pural Mg61) commonly loses weight in the following 4 steps: 1) interlayer water (70-190 °C); 2) Al-Hydroxides (190-280 °C); 3) Mg-Hydroxides (280-405 °C); 4) $CO_2$ (405-580 °C) (Yang et al. (2002). A study by in situ techniques of the thermal evolution of the structure of a Mg-Al-CO3 layered double hydroxide, Chemical Engineering Science 57 2945 - 2953). The remaining residue is a solid solution of MgO and $Al_2O_3$. Cutinase loses weight almost immediately, because of the residual moisture. By observing $T_D^{20}$ data of cutinase and the LDHs, the improved thermal stability is evident. The sample with the lower residue is LDH/Cut-2.2/1, coherent with a higher organic loading (Table 3).

## 2.2 Retaining activity into the inorganic host

The activity was tested on LDH/Cut-2.2/1 powder (in dry form, not subjected to a release) finding a negligible activity recovery (around 0.3% respect to the total activity of cutinase added in the initial immobilization medium). This suggests that the specific interactions between cutinase and LDH layers (non-covalent interactions, such as hydrogen bonds, Van der Waals forces, hydrophobic affinity, and ionic exchanges) may have induced some reversible alterations in the three-dimensional structure of the enzyme molecules, with their catalytic triad becoming less easily available to the substrate (Frey et al. (2010). Immobilization of the aminopeptidase from Aeromonas proteolytica on Mg2+/Al3+ layered double hydroxide particles. ACS applied materials & interfaces, 2(10), 2828-2832).

Therefore, the inorganic LDH structure increases the thermal stability of the enzyme, protects its activity, which remains dormant while immobilized in the inorganic lamellae.

## 2.3 Release properties

Once immobilized in LDH host structure, the enzyme must be activated to exert its degrading activity, therefore, kinetic release studies were carried out to evaluate its residual activity. The triggering occurs through an ion exchange mechanism in salt solutions: the anions from the salt interact with the positive charged lamellae of the host structure and substitute the anionic protein, which can be released and recover its degrading activity.

A study is carried out on LDH/Cut-2.2/1, in $Na_2SO_4$ aqueous solution (0.08M, pH 7.5). The recovery after release was evaluated in terms of protein amount and activity and the results after 12 h are showed in **Table 2.** Most of the activity is recovered in the first 15 minutes (around 37% in $Na_2SO_4$ aqueous solution) and the plateau is reached in almost 3-4 h. After 12 h, the total activity recovery is 67% in sulfate solution. Moreover, by comparing the protein with activity results, the data are in almost perfect agreement and there is no significant loss of enzyme activity (less than 2% respect to the released protein amount) indicating that the enzyme mostly returns to its original conformation and activity after the release from the LDH host structure.

Similar tests were conducted for all the other samples (in sulfate solution) and the data are reported in **Table 2.** As already mentioned in the previous paragraph, the immobilization efficiency is high and constant up to an enzyme loading = 2.2/1, but concerning the protein released, the values obtained decrease above and below such enzyme loading ratio. It seems that such loading is the optimum balance in terms of immobilization efficiency and higher protein released.

After such preliminary trial, release tests in different media were carried out along 4 h: sodium phosphate and citrate-phosphate buffers, potassium carbonate, sodium chloride were chosen and compared to water and sodium sulfate. The data are summarized in **Table 4.** Between 60% and 70% of the enzyme is recovered in phosphate and citrate-phosphate buffers, potassium carbonate, and sodium sulfate. In all cases, the activity retention of the enzyme is 100%. A negligible release in NaCl is found, as well as in water, confirming the previous trial.

Following to such data, sodium phosphate buffer was chosen as release media for degradation experiments of formulated polymeric samples, because of the affinity of phosphate anions for the LDH structure, and the fact that phosphate buffer is also the optimal medium for the activity of cutinase towards the degradation of several polyesters.

**Table 4.** Protein and activity recovery of cutinase from LDH/Cut-2/1 after 4 h release in different solutions.

| Sample | Release solution | Protein recovery (%) | Activity recovery (%) | Retention activity of released proteins (%) |
|---|---|---|---|---|
| LDH/Cut-2.2/1 | Buffer phosphate | 59 ± 1 | 59 ± 2 | 100 |
| | Buffer citrate-phosphate | 69 ± 1 | 70 ± 1 | 100 |
| | $Na_2SO_4$ | 57 ± 3 | 59 ± 0 | 100 |
| | $K_2CO_3$ | 69 ± 1 | 69 ± 0 | 100 |
| | NaCl | 4 ± 0 | 2 ± 0 | 50 |
| | $H_2O$ | 10 ± 0 | 10 ± 1 | 100 |

### 2.4 Thermal Resistance

The native Cutinase, exhibits an optimal reaction temperature between 35 and 70 °C. In order to investigate the thermal resistance of free enzyme, the half-life (time at which the enzyme activity is reduced to a half of the original one) of the native Cutinase was calculated at 90 °C, i.e., temperature higher than that included in its optimal reaction range and, at the same time, below the boiling point of the buffer solution. By considering the residual activity over time a half-life of 22 min ($\pm$ 0.54) at 90 °C was estimated for the free Cutinase by fitting the residual activity data with a second order kinetic ($r^2 = 0.99$).

On the other hand, the half-life of the LDH-immobilized Cutinase at 90 °C reached up to 136 min, therefore, the thermal resistance of the Cutinase increases 6 times thanks to its immobilization on MgAl-LDH support.

Simultaneously, other thermal treatments were carried out on LDH/Cut-2.2/1 powder by subjecting it to high temperatures in order to simulate industrial processes (such as dry/wet lamination or film extrusion). The residual activity respect to the control sample (assumed as 100%) resulted very high. In particular, a negligible activity loss occurred after incubation of the LDH-immobilized Cutinase at 70 °C (for 35 seconds), while more than 60% of enzyme residual activity was measured after the treatment at 105 or 150 °C (both followed by 200 °C for 10 seconds) **(Table 5).**

Therefore, the protecting role of the inorganic LDH structure is confirmed, also in extreme conditions, namely after thermal treatments at high temperatures.

**Table 5**: Test conditions of thermal resistance experiments.

| Sample | Conditions Temperature (°C) and Time (sec) | Residual activity upon 4 h (respect to the control) (%) |
|---|---|---|
| LDH/Cut-2.2/1 | ambient | 100 |
| | 70 °C (35 sec) | 96 |
| | 105 °C (180 sec) + 200 °C (10 sec) | 67 |
| | 150 °C (180 sec) + 200 °C (10 sec) | 63 |

**2.5 Evaluation of enzymatic degradation of poly(butylene succinate-co-adipate (PBSA) formulated with LDH-immobilized cutinase**

*2.5.1 Test upon 2 h with different loading of LDH/Cut-2.2/1 dispersed in PBSA*

The enzymatic degradation of PBSA formulated with different percentage of LDH-protected cutinase (i.e., 1, 2.5 and 5 wt%) was investigated in order to evaluate the lower amount able to guarantee the degradation and thus optimize the polymeric formulation system at lab-scale for PBSA. The LDH-protected cutinase used was the best in terms of immobilization efficiency and enzyme release, i.e. LDH/Cut-2.2/1.

PBSA films formulated with LDH-protected cutinase were efficiently and rapidly degraded when incubated in sodium phosphate buffer (0.1 M) at 40 °C. It can be noted from **Table 6** that for each sample, the degradation increases according to the amount of LDH-Cutinase loading, i.e. at 60 min the degradation of PBSA-LDH/Cut-2.2/1-1% is 34%, with PBSA-LDH/Cut-2.2/1-2.5% is 58%, with PBSA-LDH/Cut-2.2/1-5% is 68%.

The data related to the protein concentration (mg/mL) and the enzymatic activity (U/mL) measured in the buffer after 120 min of incubation are reported in **Table 7.** In all tests the amount and activity of cutinase released in sodium phosphate buffer increase over time and the LDH-protected cutinase is able to degrade PBSA, even at 1 wt%.

**Table 6:** Degradation (%) of PBSA formulated with different percentages of LDH/Cut-2.2/1 after 1 h and 2 h of incubation in sodium phosphate buffer 0.1 M.

| Sample film | Degradation after 1 h, 40 °C, phosphate buffer (%) | Degradation after 2 h, 40 °C, phosphate buffer (%) |
|---|---|---|
| PBSA-LDH/Cut-2.2/1-1% | 33.6 | 78.3 |
| PBSA-LDH/Cut-2.2/1-2.5% | 57.7 | 85.0 |
| PBSA-LDH/Cut-2.2/1-5% | 68.1 | 97.6 |

**Table 7:** Protein concentration (mg/mL) and enzymatic activity (U/mL) measured in buffer after 2 h incubation of PBSA formulated with different percentages of LDH/Cut-2.2/1, and the respective release (%) of protein and activity.

| Sample | Protein concentration (mg/mL) | Protein release (%) | Enzymatic activity (U/mL) | Enzymatic activity release (%) |
|---|---|---|---|---|
| PBSA-LDH/Cut-2.2/1-1% | 0.02 ± 0.00 | 34.2 ± 0.3 | 6.3 ± 0.1 | 9.1 ±0.2 |
| PBSA-LDH/Cut-2.2/1-2.5% | 0.05 ± 0.01 | 28.8 ± 3.3 | 18.1±0.4 | 10.4±0.2 |
| PBSA-LDH/Cut-2.2/1-5% | 0.07 ± 0.00 | 19.2 ± 0.4 | 35.1 ± 1.3 | 10.2 ± 0.4 |

**2.6 Evaluation of enzymatic degradation of a polyurethane adhesive formulation with LDH-immobilized cutinase**

*2.6.1 Test upon 48 h with 1 and 5 wt% of LDH/Cut-2/1$_{surf}$ dispersed in polyurethane-based adhesive*

The enzymatic degradation of a formulated polyurethane with two different percentages of LDH-immobilized cutinase (PUR-SAMPLE 62-8, PUR-SAMPLE 62-2 respectively with 1% and 5%, prepared as described under 1.7) was investigated via weight loss tests The reference was the same formulation without any LDH (PUR-SAMPLE 62-1, comparative example from 1.7). In order to improve the dispersion of LDH/Cut in polyurethane, the sample used for the formulation was subjected to a supplementary surface treatment with sodium stearate as anionic surfactant.

The results highlighted a negligible weight loss in the reference sample PUR-SAMPLE 62-1 without the LDH/Cut-2/1$_{surf}$. On the other hand, PUR-SAMPLE 62-2 degraded 54.9% after 24 h of incubation in sodium phosphate buffer (Table 8), and 57.2 wt% after 48 h (not shown), while PUR-SAMPLE 62-8 lost 41.0% of its weight after 24 h of incubation and 47.2% after 48 h. This suggests that the enzyme can be released from LDH system and can rapidly degrade the adhesive. The samples, kept at TA under vacuum for 24 h, did not decrease their initial weight, highlighting the fact that the adhesive is not degraded until the enzyme is triggered.

**Table 8**: Degradation (%) of polyurethane formulations after 24 hours of incubation under vacuum or in sodium phosphate buffer 0.1 M.

| Sample film | LDH/Cut-2.2/1$_{surf}$ (wt%) | Degradation after 24h, TA, vacuum (%) | Degradation after 24h, 40°C, phosphate buffer (%) |
|---|---|---|---|
| PUR-SAMPLE 62-1 | 0 | 0.0 $\pm$ 0.0 | 2.1 $\pm$ 0.0 |
| PUR-SAMPLE 62-8 | 1 | 0.0 $\pm$ 0.0 | 41.0 $\pm$ 1.1 |
| PUR-SAMPLE 62-2 | 5 | 0.0 $\pm$ 0.0 | 54.9 $\pm$ 3.2 |

**Claims**

1. Carboxylic ester hydrolase of the enzyme class EC 3.1.1.74 (cutinases) or EC 3.1.1.3 (triacylglycerol lipases) immobilized in a layered double hydroxide structure (LDH).

2. The immobilized carboxylic ester hydrolase according to claim 1, wherein the LDH comprises at least one cation selected from the group consisting of $Mg^{2+}$, $Ni^{2+}$, $Zn^{2+}$, $Co^{2+}$, $Ca^{2+}$ and $Cu^{2+}$ and at least cation selected from the group consisting of $Al^{3+}$, $Ga^{3+}$, $Fe^{3+}$ and $Cr^{3+}$.

3. The immobilized carboxylic ester hydrolase according to claim 2, wherein the LDH comprises $Mg^{2+}$ and $Al^{3+}$.

4. The immobilized carboxylic ester hydrolase according to any one of claims 1 to 3, wherein the ratio of carboxylic ester hydrolase to trivalent cations is between 0.5 and 2.2.

5. The immobilized carboxylic ester hydrolase according to any one of claims 1 to 4, wherein the surface of the particles formed by the immobilized carboxylic ester hydrolase is coated by surfactant.

6. Polymer comprising an immobilized carboxylic ester hydrolase as defined by any one of claims 1 to 5, wherein the polymer comprises ester groups.

7. Method comprising the steps of

   a1) Providing an immobilized carboxylic ester hydrolase as defined by any one of claims 1 to 5 and a polymer having ester groups; and
   b1) Mixing the polymer having ester groups and the immobilized carboxylic ester hydrolase, so that a polymer comprising the immobilized carboxylic ester hydrolase is formed;
   or
   a2) Providing a crosslinkable composition comprising (i) a prepolymer containing carboxylic ester groups and either isocyanate groups or groups that can react with NCO groups and (ii) the immobilized carboxylic ester hydrolase as defined by any one of claims 1 to 5; and
   b2) crosslinking the prepolymer (i) with a crosslinker containing either isocyanate-reactive groups or isocyanate-groups so that a polymer is formed from the polymerizable composition and said polymer comprises the immobilized carboxylic ester hydrolase.

8. The method according to claim 7, further comprising a subsequent method step c) of activating the immobilized carboxylic ester hydrolase so that it splits at least some of the ester bonds of the polymer.

9. Polymer obtained or obtainable by the method according to claim 7.

**10.** The polymer according to claim 6 or 9, wherein the polymer is a polyester polyurethane or a polyester.

**11.** Laminate comprising the polymer according to claim 6 or 9.

**12.** Use of the immobilized carboxylic ester hydrolase as defined by any one of claims 1 to 5 for the degradation of a polymer comprising ester-groups.

**13.** Kit of parts comprising

a) An immobilized carboxylic ester hydrolase as defined by anyone of claims 1 to 5; and
b)

(i) in one container a prepolymer containing carboxylic ester groups and additionally either isocyanate groups or groups that can react with NCO and in a second container a crosslinker containing either isocyanate-reactive groups or isocyanate-groups or
(ii) a polymer having ester groups.

**14.** Use of the kit according to claim 13 for manufacturing an enzymatically degradable polymer.

**EP 4 321 616 A1**

<table>
<tr><td colspan="3" style="text-align:center"><strong>EUROPEAN SEARCH REPORT</strong></td><td><strong>Application Number</strong><br>EP 22 18 9534</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AGHAEI H. ET AL: "Utilization of two modified layered doubled hydroxides as supports for immobilization of Candida rugosa lipase", INTERNATIONAL JOURNAL OF BIOLOGICAL MACROMOLECULES, vol. 162, 18 June 2020 (2020-06-18), pages 74-83, XP086280591, ISSN: 0141-8130, DOI: 10.1016/J.IJBIOMAC.2020.06.145 [retrieved on 2020-06-18] | 1-5 | INV.<br>C12N9/18<br>C12N9/20<br>C12N11/093<br>C12N11/096<br>C12N9/96<br>C08J11/10<br>C12N11/14 |
| Y | * the whole document * | 6,7,9-14 | |
| A | * abstract * | 8 | |
| | ----- | | |
| X | RAHMAN M. B. A. ET AL: "Enzymatic synthesis of methyl adipate ester using lipase from Candida rugosa immobilised on Mg, Zn and Ni of layered double hydroxides (LDHs)", JOURNAL OF MOLECULAR CATALYSIS B : ENZYMATIC,, vol. 50, no. 1, 21 November 2007 (2007-11-21), pages 33-39, XP022356531, ISSN: 1381-1177 | 1-4,12 | |
| | | | **TECHNICAL FIELDS SEARCHED (IPC)** |
| | | | C12N<br>C12R<br>C09J |
| Y | * the whole document * | 6,7,9-14 | C08J |
| A | * abstract * | 5,8 | |
| | ----- | | |
| | -/-- | | |

The present search report has been drawn up for all claims

| Place of search | Date of completion of the search | Examiner |
|---|---|---|
| The Hague | 19 January 2023 | van de Kamp, Mart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 4

Europäisches
Patentamt

European
Patent Office

Office européen
des brevets

## EUROPEAN SEARCH REPORT

Application Number

EP 22 18 9534

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RAHMAN M. B. A. ET AL: "Immobilisation of lipase from Candida rugosa on layered double hydroxides of Mg/Al and its nanocomposite as biocatalyst for the synthesis of ester", CATALYSIS TODAY, vol. 93-95, 1 September 2004 (2004-09-01), pages 405-410, XP027186050, ISSN: 0920-5861 [retrieved on 2004-09-16] | 1-5 | |
| Y | * the whole document * | 6,7,9-14 | |
| A | * abstract * | 8 | |
| X | SILVA DIAS G. ET AL: "Immobilization of Pseudomonas cepacia lipase on layered double hydroxide of Zn/Al-Cl for kinetic resolution of rac-1-phenylethanol", ENZYME AND MICROBIAL TECHNOLOGY, vol. 130, 109365, 21 June 2019 (2019-06-21), pages 1-9, XP085774119, ISSN: 0141-0229, DOI: 10.1016/J.ENZMICTEC.2019.109365 [retrieved on 2019-06-21] | 1,2,4,12 | |
| | | | TECHNICAL FIELDS SEARCHED (IPC) |
| Y | * the whole document * | 6,7,9-14 | |
| A | * abstract * | 3,5,8 | |
| X | RAHMAN M. B. A. ET AL: "Immobilization of Lipase From Candida rugosa on Layered Double Hydroxides for Esterification Reaction Introduction", APPLIED BIOCHEMISTRY AND BIOTECHNOLOGY, vol. 118, 1 July 2004 (2004-07-01), pages 313-320, XP093014760, | 1,2,4 | |
| Y | * the whole document * | 6,7,9-14 | |
| A | * abstract * | 3,5,8 | |

-/--

The present search report has been drawn up for all claims

| Place of search | Date of completion of the search | Examiner |
|---|---|---|
| The Hague | 19 January 2023 | van de Kamp, Mart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 4

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 03/100052 A1 (UNIV BEIJING CHEMICAL [CN]) 4 December 2003 (2003-12-04) | 1-4 | |
| A | * the whole document * <br> * paragraph [0018] * <br> * claim 3 * | 5,12 | |
| Y,D | WO 2019/043145 A1 (CARBIOS [FR]) 7 March 2019 (2019-03-07) | 6,7,9-14 | |
| A | * the whole document * | 1-5,8 | |
| Y,D | WO 2016/198652 A1 (CARBIOS [FR]) 15 December 2016 (2016-12-15) | 6,7,9-14 | |
| A | * the whole document * | 1-5,8 | |
| Y,D | WO 2019/043134 A1 (CARBIOLICE [FR]) 7 March 2019 (2019-03-07) | 6,7,9-14 | |
| A | * the whole document * | 1-5,8 | |
| Y | CN 114 262 723 A (UNIV TIANJIN) 1 April 2022 (2022-04-01) | 6,7,9-14 | |
| A | * the whole document * | 1-5,8 | TECHNICAL FIELDS SEARCHED (IPC) |
| Y | MOHANAN N. ET AL: "Microbial and Enzymatic Degradation of Synthetic Plastics", FRONTIERS IN MICROBIOLOGY, vol. 11, 580709, 26 November 2020 (2020-11-26), pages 1-22, XP055919145, DOI: 10.3389/fmicb.2020.580709 | 6,7,9-14 | |
| A | * the whole document * | 1-5,8 | |

-/--

The present search report has been drawn up for all claims

| Place of search | Date of completion of the search | Examiner |
|---|---|---|
| The Hague | 19 January 2023 | van de Kamp, Mart |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 3 of 4

Europäisches
Patentamt

European
Patent Office

Office européen
des brevets

**EUROPEAN SEARCH REPORT**

Application Number

EP 22 18 9534

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GARCÍA J. L.: "Enzymatic recycling of polyethylene terephthalate through the lens of proprietary processes", MICROBIAL BIOTECHNOLOGY, vol. 15, no. 11, 20 July 2022 (2022-07-20), pages 2699-2704, XP093014811, GB ISSN: 1751-7915, DOI: 10.1111/1751-7915.14114 Retrieved from the Internet: URL:https://onlinelibrary.wiley.com/doi/full-xml/10.1111/1751-7915.14114> | 6,7,9-14 | |
| A | * the whole document * | 1-5,8 | |

----- 

TECHNICAL FIELDS
SEARCHED     (IPC)

The present search report has been drawn up for all claims

| Place of search | Date of completion of the search | Examiner |
|---|---|---|
| The Hague | 19 January 2023 | van de Kamp, Mart |

EPO FORM 1503 03.82 (P04C01)

page 4 of 4

## ANNEX TO THE EUROPEAN SEARCH REPORT
## ON EUROPEAN PATENT APPLICATION NO.

EP 22 18 9534

This annex lists the patent family members relating to the patent documents cited in the above-mentioned European search report.
The members are as contained in the European Patent Office EDP file on
The European Patent Office is in no way liable for these particulars which are merely given for the purpose of information.

19-01-2023

| Patent document cited in search report | Publication date | Patent family member(s) | Publication date |
|---|---|---|---|
| WO 03100052 A1 | 04-12-2003 | CN 1459502 A | 03-12-2003 |
| | | WO 03100052 A1 | 04-12-2003 |
| WO 2019043145 A1 | 07-03-2019 | BR 112020004083 A2 | 13-10-2020 |
| | | CA 3072865 A1 | 07-03-2019 |
| | | CN 111051394 A | 21-04-2020 |
| | | DK 3676316 T3 | 10-01-2022 |
| | | EP 3676316 A1 | 08-07-2020 |
| | | EP 3904431 A1 | 03-11-2021 |
| | | ES 2901746 T3 | 23-03-2022 |
| | | JP 2020531672 A | 05-11-2020 |
| | | US 2020190279 A1 | 18-06-2020 |
| | | US 2022227957 A1 | 21-07-2022 |
| | | WO 2019043145 A1 | 07-03-2019 |
| WO 2016198652 A1 | 15-12-2016 | BR 112017026693 A2 | 21-08-2018 |
| | | BR 112017026697 A2 | 21-08-2018 |
| | | BR 122020011682 B1 | 24-05-2022 |
| | | CA 2987705 A1 | 15-12-2016 |
| | | CA 2987842 A1 | 15-12-2016 |
| | | CN 107709457 A | 16-02-2018 |
| | | CN 107835829 A | 23-03-2018 |
| | | CN 113621223 A | 09-11-2021 |
| | | EP 3307811 A1 | 18-04-2018 |
| | | EP 3307812 A1 | 18-04-2018 |
| | | JP 6907132 B2 | 21-07-2021 |
| | | JP 6985154 B2 | 22-12-2021 |
| | | JP 2018520231 A | 26-07-2018 |
| | | JP 2018525457 A | 06-09-2018 |
| | | JP 2021119240 A | 12-08-2021 |
| | | JP 2021169608 A | 28-10-2021 |
| | | US 2018142097 A1 | 24-05-2018 |
| | | US 2018186943 A1 | 05-07-2018 |
| | | US 2020339766 A1 | 29-10-2020 |
| | | WO 2016198650 A1 | 15-12-2016 |
| | | WO 2016198652 A1 | 15-12-2016 |
| WO 2019043134 A1 | 07-03-2019 | AU 2018326547 A1 | 27-02-2020 |
| | | BR 112020004040 A2 | 01-09-2020 |
| | | CA 3073791 A1 | 07-03-2019 |
| | | CN 111278916 A | 12-06-2020 |
| | | EP 3676329 A1 | 08-07-2020 |
| | | JP 2020531671 A | 05-11-2020 |
| | | KR 20200043441 A | 27-04-2020 |
| | | US 2020199354 A1 | 25-06-2020 |
| | | WO 2019043134 A1 | 07-03-2019 |

EPO FORM P0459

For more details about this annex : see Official Journal of the European Patent Office, No. 12/82

**page 1 of 2**

## ANNEX TO THE EUROPEAN SEARCH REPORT
## ON EUROPEAN PATENT APPLICATION NO.

EP 22 18 9534

This annex lists the patent family members relating to the patent documents cited in the above-mentioned European search report.
The members are as contained in the European Patent Office EDP file on
The European Patent Office is in no way liable for these particulars which are merely given for the purpose of information.

19-01-2023

| Patent document cited in search report | Publication date | Patent family member(s) | Publication date |
|---|---|---|---|
| CN 114262723 A | 01-04-2022 | NONE | |

For more details about this annex : see Official Journal of the European Patent Office, No. 12/82

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2019225954 A
- WO 2020021116 A
- WO 2020021117 A
- WO 2020021118 A
- WO 2018109183 A
- WO 2019043134 A
- CA 3072865
- WO 2016198652 A

### Non-patent literature cited in the description

- **CAVANI et al.** *Catalysis Today,* 1991, vol. 11, 173-301
- **GAO et al.** *Langmuir,* 2018, vol. 34 (19), 5386-5395
- **LEIMENSTOLL ; STEPANSKI.** Polyurethanklebstoffe. Springer Vieweg Essentials
- **MEIER-WESTHUES et al.** Polyurethanes. 2019
- *Justus Liebigs Annalen der Chemie,* 1949, vol. 562, 75-136
- **MEIER-WESTHUES et al.** Polyurethanes. Vinzenz Hanover, 2019
- **LOWRY, O. H. ; ROSEBROUGH, N. J. ; FARR, A. L. ; RANDALL, R. J.** Protein measurement with the Folin phenol reagent. *Journal of biological chemistry,* 1951, vol. 193, 265-275
- **ZANAROLI et al.** Selection of commercial hydrolytic enzymes with potential antifouling activity in marine environments. *Enzyme and microbial technology,* 2011, vol. 49 (6-7), 574-579
- **BRUNA et al.** Assembly of nitroreductase and layered double hydroxides toward functional biohybrid materials. *Journal of colloid and interface science,* 2019, vol. 533, 71-81
- **TOTARO et al.** Dual chain extension effect and antibacterial properties of biomolecules interleaved within LDH dispersed into PBS by in situ polymerization. *Dalton Trans.,* 2018, vol. 47, 3155-3165
- **YANG et al.** A study by in situ techniques of the thermal evolution of the structure of a Mg-Al-CO3 layered double hydroxide. *Chemical Engineering Science,* 2002, vol. 57, 2945-2953
- **FREY et al.** Immobilization of the aminopeptidase from Aeromonas proteolytica on Mg2+/Al3+ layered double hydroxide particles. *ACS applied materials & interfaces,* 2010, vol. 2 (10), 2828-2832